## Europäisches Patentamt

## European Patent Office

## Office européen des brevets

(11) Publication number: **0 159 735**
**A1**

(12) **EUROPEAN PATENT APPLICATION**

(21) Application number: **85200391.2**

(22) Date of filing: **15.03.85**

(51) Int. Cl.⁴: **A 61 K 9/20,** A 61 K 31/65

(30) Priority: **22.03.84 NL 8400911**

(71) Applicant: **DAGRA N.V., Verrijn Stuartweg 60, NL-1112 AX Diemen (NL)**

(43) Date of publication of application: **30.10.85 Bulletin 85/44**

(72) Inventor: **Jauw, Tjoe Hang, Boschplaat 35, NL-1112 KR Diemen (NL)**

(84) Designated Contracting States: **AT BE CH DE FR GB IT LI LU NL SE**

(74) Representative: **Kooy, Leendert Willem et al, OCTROOIBUREAU VRIESENDORP & GAADE P.O. Box 266, NL-2501 AW The Hague (NL)**

(54) Pharmaceutical composition containing tetracyclin or doxycyclin salts and a process of preparing such a composition.

(57) Pharmaceutical composition containing tetracyclin or one or more doxycyclin salts and a solic basic substance.

EP 0 159 735 A1

0159735

PHARMACEUTCIAL COMPOSITION, CONTAINING TETRACYCLIN
OR DOXYCYCLIN SALTS

The invention relates to a pharmaceutical compositon
containing tetracyclin or doxycyclin salts.

There are various medicines which may cause a gullet
ulcer in that the administration form tends to keep
sticking in the gullet, particularly in case of an
anatomic defect. Then, a local high concentration of
medicine occurs, which results in an ulcer. In order
to avoid this it is often recommended to swallow the
medicine in standing or sitting position with a
sufficient amount of liquid. Medicines with great
ulcerous risks are amongst others tetracyclin and
doxycyclin salts, emepromium bromide, potassium
chloride, alprenolol, iron salts and various
analgetics and anti-flogistic analgetics. Solutions
of tetracyclin and doxycyclin salts have a low pH
value and this acid property is a considerable
ulcerous factor.

The problems connected herewith are paid attention to
in publications and literature articles, i.a.
in Current Therapeutic Research 34, 110 (1983),
J. Pharm. Sc. 71, 975 (1982) and Ph. Wkbl. 117, 1022
(1982).

-2-

It is usual to administer tetracyclin and doxycyclin salts in gelatin capsule form. Recently in view of the great risks of the sticking to the gullet wall various attempts have been made to prevent gullet damage.

They consist of the use of tablets as administration form, although the sticking capacity may differ dependent upon the auxiliary substances used and the size of the tablet.

Granulate or powder which is dissolved in water before being swallowed is also applied as administration form.

Finally, in particular the doxycycline is also administered as a base, which is insoluble in water.

Now the invention aims at providing a pharmaceutical composition containing tetracyclin or doxycyclin salts, which administration form makes it possible to apply the active substances in question as a medicine without the risk that the gullet ulcer will appear.

The composition in question is characterized in that a solid basic substance is incorporated therein.

If the solid administration form, either capsule, tablet or coated tablet, would keep sticking in the gullet, the tetracyclin or doxycyclin salt will react with the basis substance because of the entering of moist, in consequence of which the acidic nature is neutralized and the risk of gullet damage is prevented.

-3-

As soon as the doxycyclin, c.q. tetracyclin lands in the stomach as a free base, it is converted into the soluble form again by the acidic medium, so that the biological availability of the medicine remains unchanged. The coated tablet form as administration form is preferred to capsule or tablet, in view of the least sticking capacity in the gullet.

Preferably, a basic substance is used having a low molecular weight, so that the size of the tablet can remain as small as possible.

Some suitable basic substances are sodium hydrogen carbonate, dipotassium hydrogen phosphate, disodium hyrogen phosphate and sodium citrate.

The use of sodium hydrogen carbonte provides the additional advantage, that the bubbling effect during the neutralization of the product maintains a dispersion as fine as possible.

However, the physical separation of the dry forms of the components has to be guaranteed in order to prevent early interaction during preservation.

Some examples of a pH of a 10% solution of the doxycyclin sodium metaphosphate complex with equivalent amounts of basic substances are:

1. no addition                              PH = 2.7
2. + sodium hydrogen carbonate                   5.9
3. + dipotassium hydrogen phosphate              5.1
4. + sodium citrate                              5.2

5. + disodium hydrogen phosphate      PH = 5.5

The following example further elucidates the addition of a basic substance, in particular of sodium hydrogen carbonate.

Example

A mixture of 3.0 kg doxycyclin sodium metaphosphate complex and 0.9 amylum is granulated in ethanol in a suspended bed granulator by means of a sprinkling process with a solution of hydroxypropylmethyl cellulose. Thereupon this granulate is mixed with 0.4 kg sodium hydrogen carbonate and the granulation process is continued by sprinkling with the ethanolic solution of hydroxypropylmethyl cellulose.

The granulate thus obtained is mixed with tallow, magnesium stearate and colloidal silicon dioxide.

Herefrom tablets of 230 mgs are compressed having a content of 150 mg of doxycyclin sodium metaphosphate complex equivalent of 105 mg of doxycyclin base per tablet.

The tablets are then coated in a known manner by means of a coating suspension consisting of a sugar solution, to which a pigment, for instance titanium dioxide, has been added.

## C L A I M S

1. A process for the preparation of a pharmaceutical administration form, which contains tetracyclin or doxycyclin salts, <u>characterized</u> in that a solid basic substance is incorporated in the administration form as well.

2. A process according to claim 1, <u>characterized</u> in that sodium hydrogen carbonate is incorporated as the basic substance.

3. A pharmaceutical composition containing tetracyclin or one or more doxycyclin salts, <u>characterized</u> in that it contains a solid basic substance as well.

4. A pharmaceutical composition according to claim 3, <u>characterized</u> in that it contains sodium hydrogen carbonate.

European Patent
Office

**EUROPEAN SEARCH REPORT**

0159735

Application number

EP 85 20 0391

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 76, no. 3, 17th January 1972, page 33, no. 10405u, Columbus, Ohio, US; W.H. BARR et al.: "Decrease of tetracycline absorption in man by sodium bicarbonate" & CLIN. PHARMACOL. THER. 1971, 12(5), 779-84 * Abstract * | 1-4 | A 61 K 9/20 A 61 K 31/65 |
| | --- | | |
| Y | CHEMICAL ABSTRACTS, vol. 98, no. 9, 28th February 1983, page 445, no. 69873c, Columbus, Ohio, US; M. STARLINGER et al.: "The protective action of systemic bicarbonate ion on the gastric mucosa in shock" & WIEN, KLIN. WOCHENSCHR. 1982, 94(23), 632-6 | 1-4 | |
| | --- | | TECHNICAL FIELDS SEARCHED (Int. Cl.4) |
| Y | EP-A-0 069 097 (ASTRA LÄKEMEDEL AKTIEBOLAG) * Page 4, lines 5-7; claims * | 1-4 | A 61 K |
| | --- | | |
| Y | FR-A-2 132 856 (WEST LABORATORIES) * Claims * | 1-4 | |
| | ---     -/- | | |

The present search report has been drawn up for all claims

| Place of search THE HAGUE | Date of completion of the search 01-07-1985 | Examiner LENSEN H.W.M. |
|---|---|---|

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | CHEMICAL ABSTRACTS, vol. 101, no. 22, 26th November 1984, page 366, no. 198038p, Columbus, Ohio, US; N. SULTANA et al.: "Effect of antacids on the dissolution behavior of tetracycline and oxytetracycline" & J. PHARM. (UNIV. KARACHI) 1983, 1(2), 139-46 <br> * Abstract * | 1-4 | |
| | ----- | | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 01-07-1985 | LENSEN H.W.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03 82